# EUROPEAN PATENT APPLICATION

(11) **EP 2 522 326 A1**
(43) Date of publication of application: **14.11.2012**
(21) Application number: 12161591.8
(22) Date of filing: 27.03.2012
(51) Int. Cl.: A61F 15/00

(54) **Pilfer-proof dispenser**

(30) Priority: 10.05.2011 GB 201107753
(71) Applicant: Benedetti International Limited, Netherton, Wishaw ML2 0JG (GB)
(72) Inventor: Benedetti, Giovanni, Wishaw, ML2 0JG (GB)
(74) Representative: Coret, Sophie V.G.A.

(57) **Abstract**

The invention relates to a pilfer-proof dispenser for storing first aid items. The dispenser comprises a support and at least one cassette securely attached to a first aid item, said cassette being removably attached to said support by attachment means and being further provided with first locking means distinct from said attachment means. The dispenser further comprises a locking bar having second locking means to lock the cassette into a non-movable position on the support, said bar being moveable with respect to the support between a first position wherein said second locking means cooperates with said first locking means of said cassette to lock said cassette in said non-movable position and a second position wherein said first and second locking means do not lock said cassette. The support is further provided with means to restrict movement of the locking bar from said first position. The invention further relates to a first aid kit which includes the dispenser of the invention and to a cassette for one or more first aid items to be used in the dispenser of the invention.

## Description

### FIELD OF THE INVENTION

The present invention relates to an apparatus for holding and dispensing small first aid items such as sticking plasters, bandages, disinfecting wipes, vials of therapeutic liquids and the likes.

### BACKGROUND OF THE INVENTION

In most European countries it is a legal requirement for management to provide workplaces with a small supply of first aid items such as plasters, bandages and the likes for use by employees. Typically the first aid items supplied will be suited to the nature of the work conducted and can includes various types of plasters, wipes and bandages as well as vials of sterile saline solutions for the eyes.

Generally, therefore, the limited supply of first aid items provided will be reasonably accessible, with the injured person themselves deciding on the need for selecting and using the items. However, the provision of a limited but generally accessible supply of first aid items by an employer, is unfortunately open to abuse. This arrangement leaves open the possibility that individuals can remove a substantial amount of the whole supply of plasters for their own use in the future. Continual replenishment of stolen first aid items would clearly be an unacceptable expense. To circumvent this problem, "pilfer-proof" systems have been devised in which first aid items can only be removed one at a time, opened and/or without at least part of their wrapping. Thus first aid items can be available to those in genuine need, but the removal of multiple items for use at a later date becomes impractical.

One such system which is directed to the dispensing of plasters is described in US 6,050,413. This system consists of a series, or booklet, of wrapped plasters which is fastened by staples to a cassette along the bottom edge. The cassette is provided with a locking clip which is designed to slot into an individual compartment of a wall mounted first aid cabinet. The device further comprises a locking bar being able to lock said cassette to the device's casing into a non-removable position. The locking bar is actuated by the means of a key which can be stored away from the device.

To remove a plaster, the top portion thereof is grasped firmly and pulled away. As the bottom of the wrapper is stapled to the cassette, the wrapper is pulled apart and remains within the cassette. Advantageously the protective layer provided upon a sticking part of the plaster is stapled to the cassette and is also removed when the top portion of the plaster is pulled away, thus exposing the sticking part of the plaster which then has to be applied immediately.

### STATEMENT OF THE INVENTION

The present invention provides an alternative pilfer-proof dispenser for storing first aid items which permits greater flexibility of display.

The pilfer-proof dispenser for storing first aid items of the invention comprises:
a support;
at least one cassette securely attached to a first aid item, said cassette being removably attached to said support by attachment means and being further provided with first locking means distinct from said attachment means;
a locking bar having second locking means to lock said cassette into a non-movable position on said support, said bar being moveable with respect to the support between a first position wherein said second locking means cooperates with said first locking means of said cassette to lock said cassette in said non-movable position and a second position wherein said first and second locking means do not lock said cassette;
and wherein said support is further provided with means to restrict movement of the locking bar from said first position.

The dispenser advantageously stores multiple cassettes which may be located side by side on the support and are all locked in a non-movable position on the support when the locking bar is in the first position.

It is particularly advantageous for the removable cassette(s) to be slidably attached to the support as this enables selected cassettes to be replaced whilst allowing the succession of cassettes of various items to be rearranged without requiring the remainder to be dislodged. It is thus preferred that the attachment means of each cassette comprises a sliding part such as a hook which cooperates with a corresponding sliding groove or rail provided on or within the support.

It is also preferred that the first and second locking means includes a locking pattern which may comprise a series of interlocking projections and cavities. Advantageously the second locking means comprises a pattern which repeats itself. Advantageously the projections and cavities are provided along the length of the locking bar and can advantageously be wedge shaped.

It is further preferred that the projections are generally triangular in shape.

When combined with the feature of a sliding hook and railing this embodiment allows for each cassette to be positioned and locked by the locking bar at a great variety of positions along the rail which are specifically predetermined but which will be defined by the interlocking pattern. The more regular and small the pattern, the greater the number of positions the cassettes may take respective to one another. Thus a locking pattern made of wedge-like or triangularly shaped projections and cavities which advantageously repeats itself about each 4mm has been found to be particularly suitable to allow the cassette to be positioned anywhere along the rail within a 4mm space, while providing the locking device with the desired mechanical strength to achieve its locking function.

The invention further relates to a cassette for one or more first aid items to be used in a pilfer-proof dispenser as above described. The cassette is securely attach to said item(s), and is sized and shaped to be removably attached to the support of the pilfer-proof dispenser by attachment means. The cassette further comprises first locking means distinct from the attachment means and able to be locked in a non-movable position whilst attached to the support by a locking bar having second locking means to lock said cassette in a non-movable position on the support, the bar being moveable with respect to the support between a first position wherein the second locking means cooperates with the first locking means of said cassette to lock said cassette in a non-removable position and a second position wherein said first and second locking means does not lock said cassette.

Desirably the locking bar has two positions, so that all the cassettes located on the locking bar switch from the "locked" to the "removable" state (and vice versa) simultaneously.

A preferred option is for the removal of a cassette to require two actions, first the transition of the cassette (s) from a locked to a (re)movable state (for example by removal of the locking bar) and second for the attaching means to be disengaged from the support before the cassette can be lifted out of the device.

Advantageously the locking projections and cavities provided onto the cassette and the locking bar are evenly spaced.

The number of cassettes that can be stored in the dispenser of the invention may range from 2 to 10, preferably 4, 5 or 6.

Advantageously the first locking means of such cassette includes a locking pattern which may comprise a series of projections and cavities which cooperates, preferably in an interlocking manner, with corresponding locking projections and cavities provided on the second locking means of the locking bar.

It is also preferred that the projections and cavities are wedge-shaped, and more preferably triangular in shape.

It is further preferred that the attaching means comprises a sliding part such as a hook.

According to a particular embodiment of the invention the means to restrict movement of the locking bar from the first position comprises at least one, and preferably two keyholes which each engages with a rotating key to permit displacement of the locking bar from the first position to the second position. The means to restrict movement of the locking bar from the first position may further comprise a security cover or lid provided on said support which protects said bar from direct human access. By "direct human access" it is meant that a person cannot with only his/her bare hands easily move the locking bar to the unlocked position. The device of the invention is not designed to be a 100% security proof system but a good deterrent against occasional pilferers.

The first aid items may be held in the cassette by any convenient fastening means. For ease of removability, the item itself should not be attached to the cassette, other than via its wrapper or its lid. The item may be held in the clip by gluing, snap fitting, stapling, interference fitting or any other known methods.

The cassette support and locking bar may be made of plastic or other suitable material.

The first aid items to be dispensed can be any usual first aid items that can be found in conventional first aid kits. Conveniently these items are dispensable items of small dimensions (e.g. not in excess of 20cm², preferably 10cm²). For example, such items can be individually wrapped sticking plasters, bandages or wipes, or vials containing sterile fluids or saline solutions such as eyewash liquid.

The size and width of the cassette may be altered to suit the items held thereby. The cassette may hold a single items or a series of items. Furthermore the cassette may hold one size of first aid items such as plasters, wipes, bandages or vials. Alternatively a range of sizes and/or types of items may be held by a single cassette.

Generally, a plaster cassette according to the invention would comprise a multiplicity of plasters, preferably sterile, normally 2 to 20, more usually 6 to 15, and for example 10 or 12 plasters. By contrast, cassettes of saline solutions will only hold one vial.

The present invention further provides a first aid kit having a pilfer-proof first aid items device as described above.

The present invention will now be further described with reference to the non-limiting drawings.

### BRIEF DESCRIPTION OF THE DRAWINGS

FIG. 1 schematically illustrates a pilfer-proof dispenser according to the invention containing 6 plaster cassettes of the type shown in FIG. 3, the dispenser is shown in its normal dispensing position.
FIG. 2 shows the dispenser of FIG.1, the security cover of the pilfer-proof dispenser of FIG. 1 in an opened position, the locking means being disengaged.
FIG. 3 is a schematic illustration of a plaster cassette according to the present invention.
FIG. 4 is an enlarged and partial cross-sectional view of the dispenser of FIG.1 taken along the lines A-A'.
FIG. 5 illustrates the dispenser of FIG.1, the dispenser is shown in its closed position.
FIG. 6 is a schematic illustration of a vial cassette according to the present invention.
FIG. 7 is an enlarged and partial representation of the key/locking pin mechanism provided onto the dispenser shown in FIG. 1.

### DETAILED DESCRIPTION OF PREFERRED EMBODIMENTS OF THE INVENTION

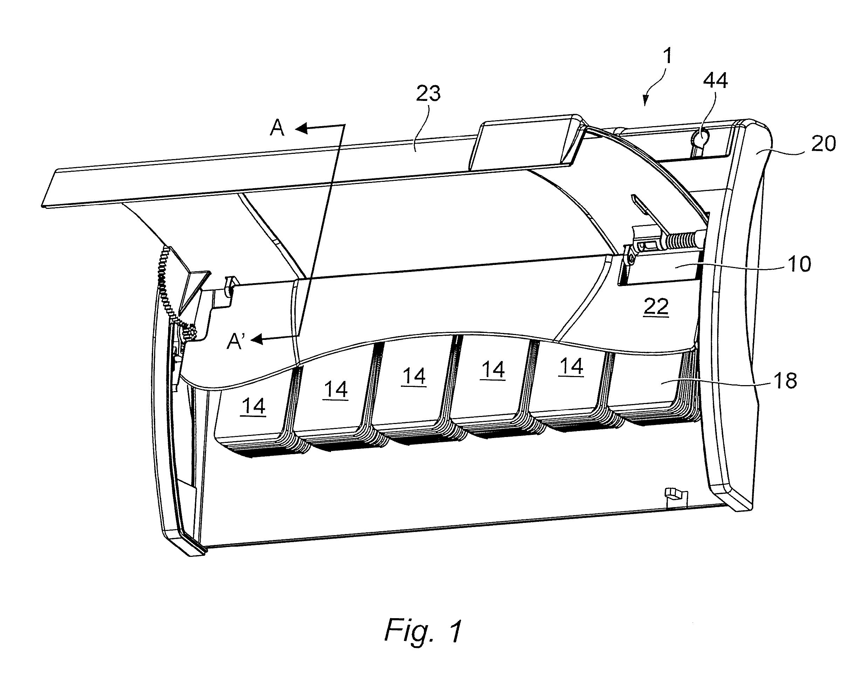

FIG. 1 illustrates a pilfer-proof dispenser 1 according to the invention containing six plaster cassettes 10 located in casing 20.

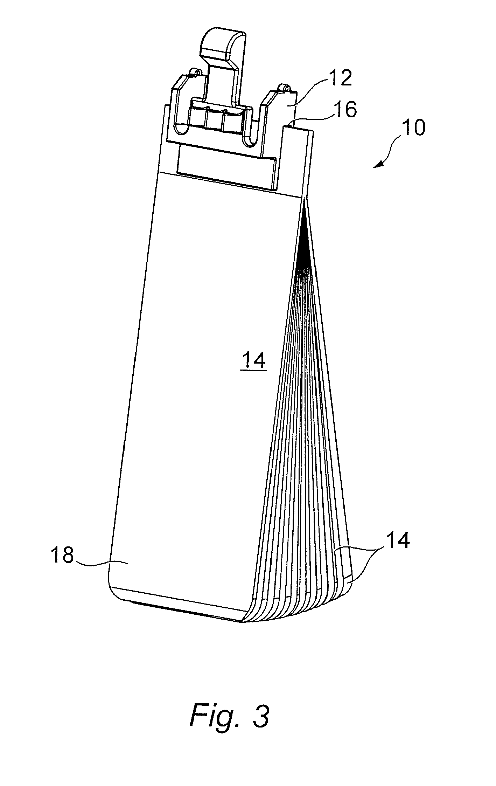

In more detail, FIG. 3 illustrates a plaster cassette 10 according to the invention. The cassette 10 comprises a plastic body 12 which holds a number (usually about 10) of wrapped plasters 14. The plaster is of the usual types and comprises an adhesive backing sheet and an absorbent dressing located approximately centrally thereon. The adhesive backing sheet is protected by cover sheets and the plaster is enclosed in a wrapper made of a suitably protective material. The wrapped plasters 14 are staked together and one of the wrappers' extremities is engaged within receiving slot or clip 16 and is secured to the body 12 by means of staples.

When a plaster is required by a user the protective lid 23, which is advantageously spring loaded, is opened by simply applying pressure on the bottom of the lid 23. Once opened, the user can access the bottom portion 18 of a plaster 14, grip it through its wrapper and pull it downwardly. The remainder of cassette 10 is attached to the pilfer-proof dispenser 1 and remains essentially stationary. The downward movement of plaster 14 causes the wrapper to tear enabling a plaster 14, held via a remaining portion of the wrapper, to be removed free of cassette 10. Since the plaster 14 is intended for imminent use, it may be desirable for at least one of the cover sheets protecting the adhesive backing sheet to be automatically peeled away from the adhesive surface as a plaster 14 is removed from the cassette 10. This can be achieved by gluing a portion of the upper cover sheet to an inner surface of the upper portion of the wrapper. Thus, when a plaster 14 is pulled out of cassette 10, the upper cover sheet remains attached to cassette 10 and approximately half of the adhesive sheet is no longer protected. The plaster 14 is thus suitable for immediate use only.

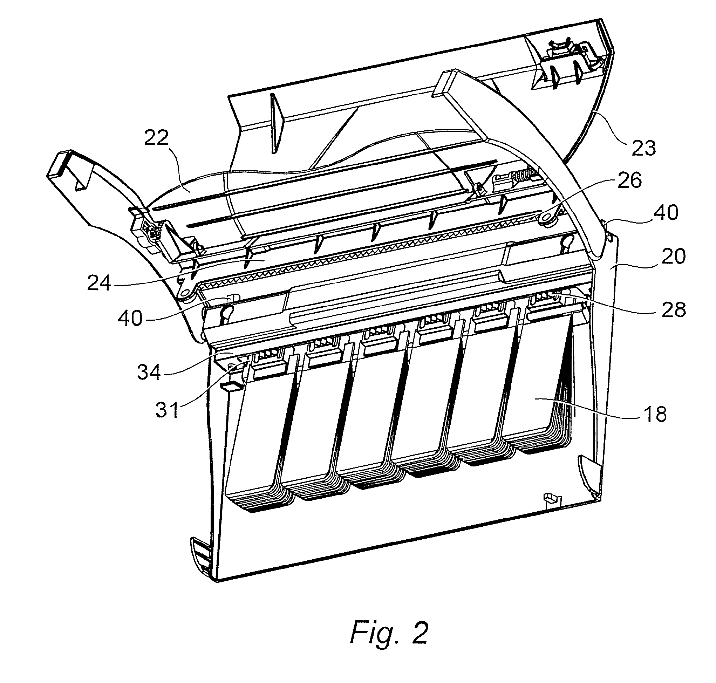

Turning back to the dispenser 1, FIG. 2 shows locking bar 24 located onto the interior of a security cover 22. As illustrated in FIG. 2, the security cover 22 is rotatably mounted onto a support such as the casing 20 and can move between an opened position (shown in FIG. 2) and a closed position (shown in FIG. 1). When the security cover 22 adopts its closed position the locking indentations 26 engage reciprocating indentations 28 provided onto cassette 10, thus locking the cassette 10 in a given position and preventing any longitudinal movements along the rail 34.

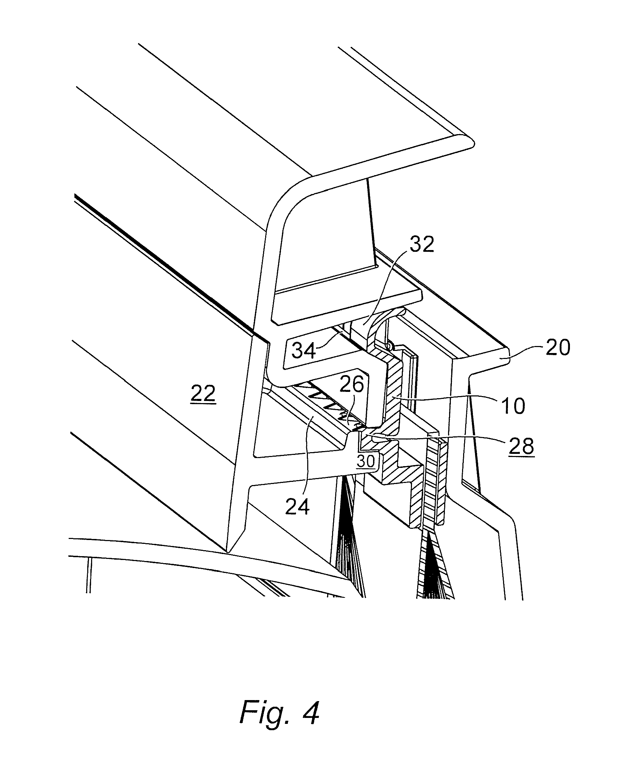

As shown in FIG.4, the locking bar 24 is provided with a protruding ledge 30 which is positioned below the indentations 28 and prevents any downward movements and/or dislodgement of the cassette 10. Each cassette 10 is located within the casing 20 by insertion through the internal longitudinal opening 31 provided thereof. Part 32 of cassette 10 is sized and shaped so that it can be fitted within the receiving sliding rail 34 provided within casing 20. Advantageously part 32 can slide along the length of rail 34 to adopt any desired position along said rail 34.

One particular advantage of the provision of wedge-shaped reciprocating indentations is that such a shape allows the cassettes to be positioned along the sliding rails at various positions which are not pre-determined. When the locking bar is moved toward the first position (i.e. closed), the cavities which are positioned on the cassette(s) will be either positioned to receive the indentations, or not. In this latter case the indentations of both the cassette and the locking bar 24 will meet. Upon a continuous force being applied by the user onto the security cover 22, the indentations on the cassettes, and thus the cassettes, themselves will be moved laterally until the indentations 26 of the locking bar 24 are positioned fully within the cavities of the cassette(s) and vice versa.

The wedge-shaped or teeth-shaped protuberances (and corresponding cavities) are particularly adapted to this self positioning feature because of their profile but various other shapes and angles could also be suitable. In particular various interlocking patterns could be provided onto cassettes 10 as there is no requirement that the locking pattern of the cassette 10 be identical to the one present on the locking bar 24. What is required is that they fit and engage each other in a locking position. Thus, only 2, or even only one, protuberance can be provided on the cassettes 10 instead of the three shown in the figures.

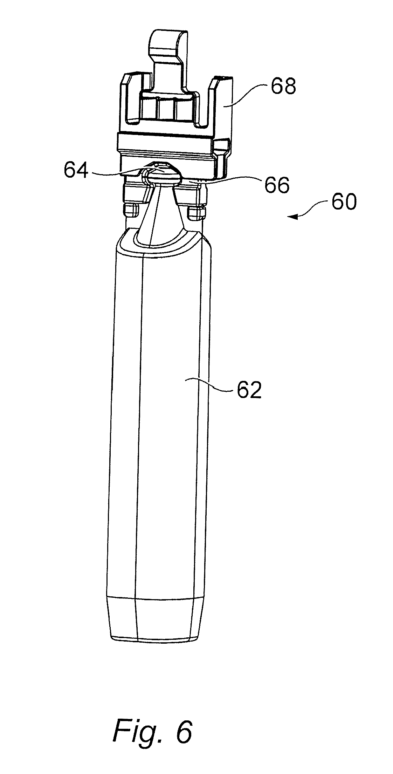

The particular self positioning feature permits to secure first aid items of varied nature and sizes therefore increasing the adaptability of the dispenser 1 while maximising the number of items (or set of items) which are displayed by the dispenser 1. In particular, items other than plasters can be displayed. For example, a vial cassette 60 holding a small vial of sterile saline or eyewash solution 62 is shown in FIG.6. The vial is made of flexible plastic material and comprises a detachable lid 64 secured to a holding tab 66. The holding tab 66 is firmly secured within the body 68 of the cassette 60 and the removal of the vial by twisting and pulling its body downward will result in its lid 64 being detached and the vial is suitable for immediate use only.

A great variety of other first aid item cassettes can be provided. Such items include bandages cassettes, antiseptic wipes cassettes etc. Most of the time, part of the wrapping of the first aid item is firmly secured to the cassette and removal of any items is accompanied by the tearing down of a portion of the wrapping, thus exposing the first aid item and necessitating its immediate use.

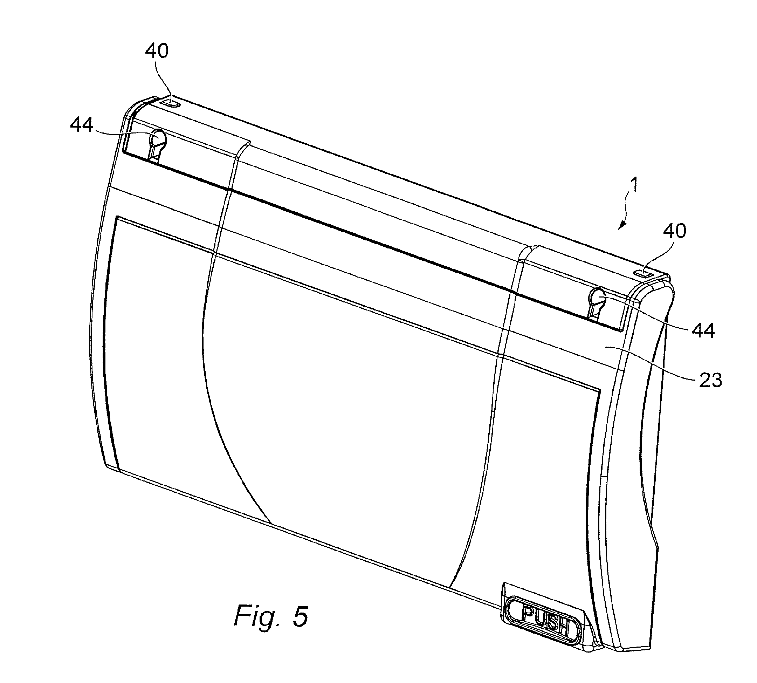

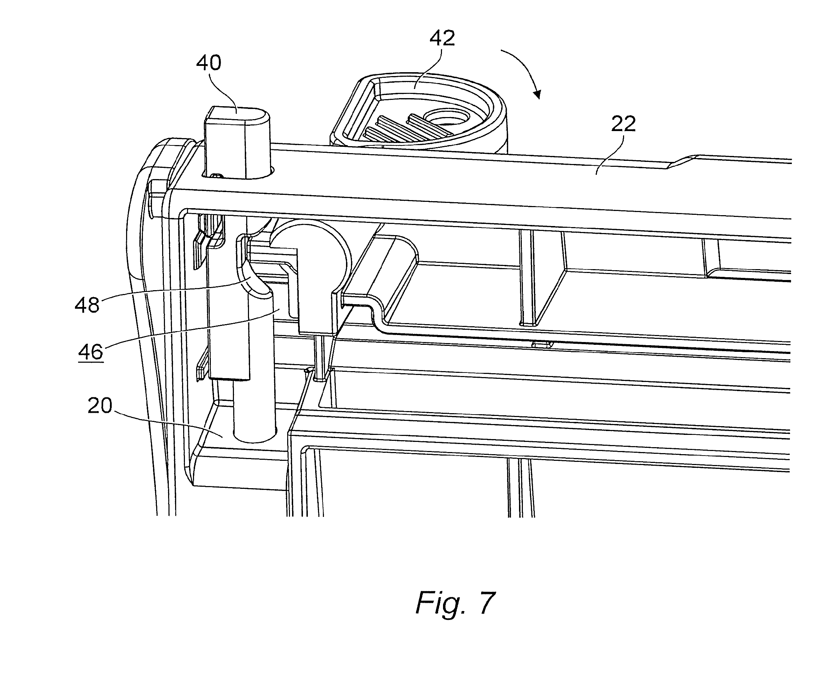

The security cover 22 (and therefore the locking bar 24) is secured into position to the casing 20 by any known suitable locking means. According to the particularly preferred embodiment shown in FIG. 7 security pins 40 provided on the top of the dispenser can be pushed down to lock the security cover 22 to the casing and prevent any rotation thereby. Advantageously the locking pins 40 are sized and shaped so that they do not protrude externally from the casing once positioned in their locking position (as shown in FIG. 5) thus preventing that they may be grabbed externally and pulled up as this would disengage the security cover 22 from the casing 20 and allow direct access to the first aid items 14.

Whilst the security cover 22 remains in its first (i.e. closed) position, as shown on FIGS. 1 and 7, the cassettes 10 cannot be removed from the casing 20. Locking bar 24 may be removed from the first position by rotating the security cover 22 and adopt the second (i.e. opened) position shown on FIG.2. To do so the security pins 40 have to be disengaged. To unlock the security pins 40 and allow the security cover 22 to adopt an open position, a key 42 is located in each of keyholes 44 and rotated, such that the extremity 46 of the key 42 may engage a corresponding indentation provided within the locking pins 40. Upon rotation of the key 42 the locking pin 40 will be urged upward and disengaged from the security cover 22. The cover 22 may therefore be moved from its first position to its second position. In its second position the locking bar 24 does not prevent the cassette 10 to be removed.

It is understood that this description is not meant to be limiting because further modifications may now suggest themselves to those skilled in the art and is intended to cover such modifications as fall within the scope of the following claims.

## Claims

1. A pilfer-proof dispenser for storing first aid items, said dispenser comprising:
a support;
at least one cassette securely attached to a first aid item, said cassette being removably attached to said support by attachment means and being further provided with first locking means distinct from said attachment means;
a locking bar having second locking means to lock said cassette into a non-movable position on said support, said bar being moveable with respect to the support between a first position wherein said second locking means cooperates with said first locking means of said cassette to lock said cassette in said non-movable position and a second position wherein said first and
second locking means do not lock said cassette;
and wherein said support is further provided with means to restrict movement of the locking bar from said first position.

2. The dispenser as claimed in claim 1, wherein multiple cassettes are located side by side on the support and are all locked in a non-movable position on said support when the locking bar is in said first position.

3. The dispenser as claimed in claim 1 or 2, wherein the attachment means of said cassette comprises a sliding part which cooperates with a corresponding sliding rail provided on the support.

4. The dispenser as claimed in any one of claims 1 to 3, wherein said first and second locking means each includes a series of interlocking projections and cavities.

5. The dispenser as claimed in claim 4, wherein said projections and cavities are provided along the length of the locking bar.

6. The dispenser as claimed in claim 4 or 5 wherein said locking projections and cavities are wedge shaped.

7. The dispenser as claimed in claim 6, wherein said projections are generally triangular in shape.

8. The dispenser as claimed in any one of claims 1 to 7, wherein said means to restrict movement of the bar from the first position comprises at least one keyhole which engages with a rotating key to permit displacement of the locking bar from the first position to the second position.

9. The dispenser as claimed in any one of claim 1 to 8, wherein said means to restrict movement of the locking bar from the first position comprises a security cover provided on said support which protects said bar from direct human access.

10. The dispenser as claimed in any one of claims 1 to 9 wherein said first aid items are individually wrapped sticking plasters, bandages or wipes or vials containing sterile fluids or saline solutions.

11. A cassette for one or more first aid items to be used in a pilfer-proof dispenser as described in any one of claims 1 to 10, said cassette being securely attached to said item, said cassette being sized and shaped to be removably attached to the support of said pilfer-proof dispenser by attachment means, said cassette further comprising first locking means distinct from said attachment means and being able to be locked in a non-movable position whilst attached to said support by a locking bar having second locking means to lock said cassette in a non-movable position on said support, said bar being moveable with respect to the support between a first position wherein said locking means cooperates with said cassette to lock said cassette in a non-removable position and a second position wherein said second locking means does not lock said cassette.

12. The cassette claimed in claim 11 wherein the first locking means of said cassette comprises a series of locking projections and cavities which cooperates with corresponding locking projections and cavities provided on said second locking means of said locking bar.

13. The cassette claimed in claim 12 wherein said projections and cavities are wedge-shaped projections and cavities.

14. The cassette claimed in any one of claims 11 to 13 wherein said attaching means comprises a sliding part.

15. A first aid kit including a pilfer-proof dispenser as claimed in any one of claims 1 to 10.
